# EUROPEAN PATENT APPLICATION

(11) **EP 2 377 879 A1**
(43) Date of publication of application: **19.10.2011**
(21) Application number: 10159951.2
(22) Date of filing: 14.04.2010
(51) Int. Cl.: C07K 14/025, A61K 39/12, C07K 19/00, C12N 15/62

(54) **N-terminal HPV E7 fusion proteins**

(71) Applicant: Deutsches Krebsforschungszentrum, 69120 Heidelberg (DE)
(72) Inventor: The designation of the inventor has not yet been filed
(74) Representative: Dick, Alexander

(57) **Abstract**

The present invention is concerned with a fusion polypeptide comprising a first domain comprising a HPV E7 polypeptide and a second domain comprising at least one peptide from a second polypeptide, wherein the first domain is located N-terminally of the second domain. It also relates to a polynucleotide coding for said fusion protein and to a vector and a host cell comprising said fusion protein and / or polynucleotide. Furthermore, the present invention is concerned with a medicament comprising the fusion protein and / or the polynucleotide and / or the vector and / or the host cell of the current invention and with a method for the manufacture of such medicament.

## Description

The present invention is concerned with a fusion polypeptide comprising a first domain comprising an HPV E7 polypeptide and a second domain comprising at least one peptide from a second polypeptide, wherein the first domain is located N-terminally of the second domain. It also relates to a polynucleotide coding for said fusion protein and to a vector and a host cell comprising said fusion protein and / or polynucleotide. Furthermore, the present invention is concerned with a medicament comprising the fusion protein and / or the polynucleotide and / or the vector and / or the host cell of the current invention and with a method for the manufacture of such medicament.

Human papillomaviruses (HPV) infect the skin or the mucosa of the anogenital and respiratory tracts, and are therefore divided into cutaneous and mucosal types. Most of them cause benign hyperproliferative lesions (warts, papilloma), although, in both groups, some types are involved in the development of malignancies. The mucosal HPV types are divided into 'low-risk' and 'high-risk'types depending on their oncogenic potential. Certain high risk types, in particular HPV 16 and HPV18, are associated with the development of a number of different malignant anogenital tumors, including cervical cancer.

Cervical cancer is the second most common cancer in women worldwide with approximately 493,000 new cases diagnosed each year and around 274,000 women dying from that disease (Parkin et al., 2006). In almost all cervical carcinomas, DNA from high risk human papilloma types can be detected (Clifford et al., 2006; Walboomers etal., 1999). In total, around 15 HPV types are known to be causative agents of the disease (Roden et al., 2006) and HPV16 is the most prevalent as it is found in more than 50% of all cervical cancers, followed by HPV18 which accounts for 20% of cases. Infection with high-risk HPV types does not necessarily lead to the development of cervical cancer, and in most cases (95%), the infections can be cleared by the immune system (Frazer, 2004; Tindle,2002).

Persistent infection with high-risk human papillomaviruses (repeated detection of the viral DNA at six month intervals), however, is a major risk factor for the development of cervical cancer and occurs in approximately 20% of infected women. Persistent infection can lead to lesions that can progress to cervical intraepithelial grade 1 lesions (CIN1). 20% of CIN1 lesions develop into CIN2 lesions, and some of these into CIN3 lesions and cervical cancer. The onset of cervical cancer is a very long process that often takes more than 10 years from the initial infection with the virus.

The main function of the early proteins E6 and E7 of papillomaviruses in the viral life cycle is the facilitation of viral replication by driving the host cell into S-phase; however, these functions can also lead to malignant transformation of the infected cell. HPV 16 E7 is a small, approximately 17 kDa, protein which is 98 amino acids long. It comprises three domains designated as CR1, CR2 and CR3. CR3 is the C-terminal region and contains two C-X-X-C zinc-binding motifs, which are known to facilitate the dimerization of E7 (Clemens et al., 1995). The intermediate CR2 domain mediates the binding of E7 to pRB, an interaction that leads to the subsequent degradation of this protein, which in turn, drives the cell into S-phase. Additionally, E7 promotes cell cycle progression by binding to cyclin A and E complexes and by inactivation of the cyclin-dependent kinase inhibitors p21CIP-1 and p27KIP-1 (Jones et al., 1997; Tommasino et al., 1993; Zerfass-Thome et al., 1996). Both E6 and E7 oncoproteins are consistently expressed in HPV-induced anogenital tumors. Their functions lead to the abrogation of cell-cycle control and apoptosis mechanisms.

The late HPV proteins L1 and L2 are structural proteins that build the icosahedral viral capsid, which is between 55 and 60 nm in diameter. The major capsid protein L1 assembles in the cytoplasm into pentamers, also called capsomeres, which are subsequently transported into the nucleus.

The L1 protein is highly conserved among papillomaviruses (de Villiers et al., 2004). HPV16 L1 consists of 505 amino acids and has a molecular weight of 55 kDa. Recombinant expression of the L1 protein (with or without L2) leads to efficient self-assembly into socalled virus-like particles (VLPs) (Kirnbauer et al., 1993). These resemble the native virion in size and shape, but do not carry viral DNA. In numerous pre-clinical studies it was shown that VLPs are highly immunogenic and elicit high titers of neutralizing antibodies, which are a prerequisite for the prevention of infection (Christensen et al., 1994; Cook et al., 1999; Kirnbauer et al., 1992). These data resulted in the industrial development of VLP-based vaccines and finally in the recent introduction of the two prophylactic formulations CervarixTM and Gardasil®. Gardasil® includes VLPs of HPV types 6, 11, 16 and 18, which are expressed in and purified from *S.cerevisiae.* CervarixTM consists of only HPV types 16 and 18. The VLPs are produced in insect cells (Trichoplusia ni (Hi-5)) infected with recombinant baculoviruses. Although both vaccines are known to induce L1-specific cellular immune responses, no therapeutic effect against existent infections or lesions could be observed (Pinto et al., 2003).

Although the introduction of the VLP-based vaccines is a significant step towards eradicating cervical cancer, there remain a number of issues to be resolved. More than 80% of all cervical cancer cases occur in resource-poor countries. However, the high production costs of the VLP vaccines limit the extent to which they can be made available for these countries. Another consideration is that the VLP vaccines only protect against infection with HPV16 and 18, which are responsible for 70% of the cancer cases, with the remaining 30% of cases caused by 12 other high-risk types. Moreover, the vaccines are strictly prophylactic, which means that women who are already infected with the virus will not benefit from vaccination.

The technical problem underlying the present invention, thus, could be seen as the provision of means and methods for complying with the aforementioned needs. The said technical problem is solved by the embodiments characterized in the claims and herein below.

Thus, the present invention relates to a fusion polypeptide comprising a) a first domain comprising a HPV E7 peptide and b) a second domain comprising at least one peptide from a second polypeptide, wherein the first domain is located N-terminally of the second domain.

As used herein, the term "domain" relates to a peptide or polypeptide comprising a sequence of at least 10, at least 20, at least 30, at least 40, or at least 50 consecutive amino acids identical to the amino acid sequence of a naturally occurring or of an artificial polypeptide. Thus, a "domain comprising a peptide" is a domain comprising a sequence of consecutive amino acids identical to the amino acid sequence of said peptide.

The term "HPV E7 peptide" relates to a peptide comprising a sequence of an HPV E7 polypeptide or parts thereof of between 50 to 150 amino acids in length, having the function of inducing an anti-HPV E7 immune response in a suitable host, e.g. a mammal. Preferably, an HPV E7 peptide comprises a sequence of a high-risk HPV E7 polypeptide or parts thereof. High-risk HPV genotypes are well known in the art and include HPV 16, 18, 31, 33, 35, 39, 45, 51, 52, 56, 58, 59, 68, 73 and 8. More preferably, the HPV E7 peptide comprises a sequence of the HPV16 E7 protein (SEQ ID NO: 1, Genbank Acc. No: ACR25131.1 GI:237863011) or parts thereof.

Also included are variants of said HPV E7 peptides, said variants having an amino acid sequence being at least 40%, at least 50%, at least 60%, at least 70%, at least 80%, at least 90%, at least 95%, least 96%, at least 97%, at least 98% or at least 99% identical to the amino acid sequence of the HPV E7 peptide and retaining the function of the HPV E7 peptides of the current invention. The percent identity values are, preferably, calculated over the entire amino acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981)), which are part of the GCG software packet (Genetics Computer Group, 575 Science Drive, Madison, Wisconsin, USA 53711 (1991)), are to be used.

In the context of the current invention, the term "fusion polypeptide" relates to a polypeptide of between 50 to 1000 amino acids in length comprising at least two different domains, the N-terminal domain being a domain comprising a HPV E7 peptide. Thus, the domain comprising a HPV E7 peptide will be read before the second domain if the amino acid sequence of the fusion protein is read from the N-terminal amino acid to the C-terminal amino acid. The polypeptide of the present invention can be recombinantly manufactured or may be chemically synthesised. The fusion polypeptide may comprise further amino acids which may serve as a tag for purification or detection or as a linker.

In a preferred embodiment of the fusion polypeptide of the present invention, said fusion polypeptide further comprises a detectable tag. The term "detectable tag" refers to a stretch of amino acids which are added to or introduced into the fusion polypeptide of the invention. Preferably, the tag shall be added C- or N- terminally to the fusion polypeptide of the present invention. The said stretch of amino acids shall allow for detection of the fusion polypeptide by an antibody which specifically recognizes the tag or it shall allow for forming a functional conformation, such as a chelator or it shall allow for visualization by fluorescent tags. Preferred tags are the Myc-tag, FLAG-tag, 6-His-tag, HA-tag, GST-tag or GFP-tag. These tags are all well known in the art.

The term fusion polypeptide also includes chemically modified fusion polypeptides, e.g., fusion polypeptides containing modified amino acids or fusion polypeptides which are, e.g., biotinylated, or are coupled to fluorophores, such as fluorescin, or Cy 3, are conformationally restricted, e.g. by disulfide bridging or by stapling (Walensky 2004, Science 305(5689): 1466-1470), or are linked to cell penetration polypeptides or protein transduction domains (Snyder 2004, Pharm Res 21(3): 389-393). Such modifications may improve the biological properties of the fusion polypeptides, e.g., cell penetration, binding, stability, or may be used as detection labels.

The variant or modified fusion polypeptides, preferably, retain the biological activity of the HPV E7 peptides. The conservation of activity can be tested by the one skilled in the art, e.g. by testing if the variant is able induce anti-HPV E7 antibodies in a suitable host.

The "second polypeptide" of the current invention is a naturally occurring or artificial polypeptide. Preferably, the second polypeptide is an HPV L1 polypeptide. As used herein, the term "HPV L1 polypeptide" relates to a peptide comprising a sequence of an HPV L1 polypeptide or parts thereof of between 25 to 550 amino acids in length, having the function of inducing anti-HPV L1 antibodies in a suitable host, e.g. a mammal. Preferably, an HPV L1 peptide comprises a sequence of a high-risk HPV L1 polypeptide or parts thereof. More preferably, the HPV L1 peptide comprises a sequence of the HPV-16 L1 protein (SEQ ID NO: 2, Genbank Acc. No: ACN91182.1 GI:225423352) or parts thereof.

Also included are variants of said HPV L1 polypeptides, said variants having an amino acid sequence being at least 40%, at least 50%, at least 60%, at least 70%, at least 80%, at least 90%, at least 95%, least 96%, at least 97%, at least 98% or at least 99% identical to the amino acid sequence of the HPV L1 peptide and retaining the function of the HPV L1 peptides of the current invention. The percent identity values are, preferably, calculated as specified herein above. The variant or modified HPV L1 polypeptides, preferably, retain the biological activity of the HPV L1 peptides of inducing an anti HPV L1 immune response in a suitable host, e.g. a mammal. The conservation of activity can, e.g., be tested by the one skilled in the art by testing if the variant is able induce anti-HPV L1 antibodies in a suitable host.

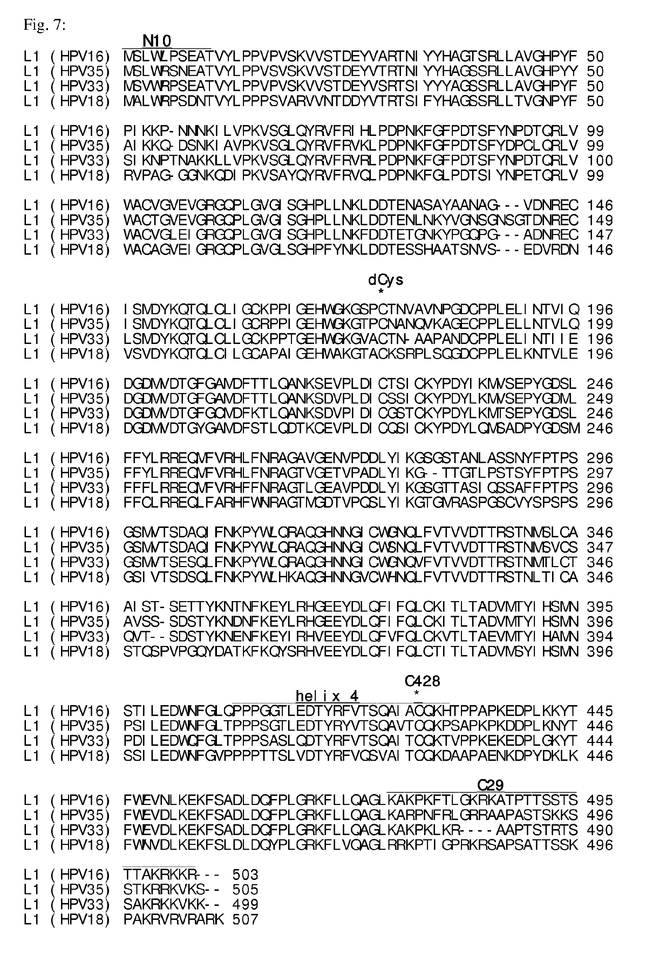

It is also contemplated by the current invention that the HPV L1 polypeptide, preferably, is a mutated HPV L1 protein comprising a deletion of the amino acids corresponding to amino acids 1 -10 of the HPV16 L1 protein and / or a deletion of the amino acids corresponding to amino acids 408 to 431 (helix 4) of the HPV 16 L1 protein. The amino acids corresponding to the respective amino acids in the HPV16 L1 protein can be determined by aligning the amino acid sequence of the L1 protein of the HPV genotype of interest to the amino acid sequence of the HPV16 L1 protein. Said alignment can be accomplished by using alignment software well known to the skilled artisan, e.g. the ClustalW multiple sequence alignment program (Larkin et al. (2007) "Clustal W and Clustal X version 2.0 " Bioinformatics, Volume: 23 (Issue: 21), 2947-2948). For an example, see e.g. Fig. 7 for an alignment of L1 amino acid sequences of HPV genotypes 16, 18, 33, and 35. More preferably, the HPV L1 polypeptide is an HPV16 L1 polypeptide comprising a deletion of the first 10 N-terminal amino acids and /or -29-32 amino acids of the C-Terminus and / or a deletion of helix 4 and / or at least one mutation of a cysteine residue forming a disulfide bond in an HPV capsid, said cysteine residue most preferably beinf the cysteine residue corresponding to the cysteine residue at position 175 in the amino acid sequence of the HPV 16 L1 protein. The amino acids corresponding to said regions or amino acids of the HPV16 L1 protein can be determined as described above (Fig. 7).

The term "second domain", preferably, relates to a domain comprising a peptide from an HPV L1 polypeptide as described herein above. Preferably, said peptide comprised in the second domain is a peptide immunogenic to a suitable host, preferably a mammal, most preferably a human. Preferably, said peptide is an HPV L1 peptide of a high-risk HPV L1 polypeptide. More preferably, the HPV L1 peptide comprises a sequence of the HPV16 L1 protein or parts thereof.

The mutations given above shall apply, mutatis mutandis, to the following:
In another preferred embodiment, the current invention relates to a polynucleotide comprising a sequence encoding the fusion polypeptide of the current invention.
The term "polynucleotide" as used in accordance with the present invention relates to a polynucleotide comprising a nucleic acid sequence which encodes the fusion polypeptide of the current invention as specified herein above. It is to be understood that a polypeptide having a specific amino acid sequence may, due to the degeneration of the genetic code, be encoded by various polynucleotides.
The polynucleotides of the present invention either essentially consist of the aforementioned nucleic acid sequences or comprise the aforementioned nucleic acid sequences. Thus, they may contain further nucleic acid sequences as well.
The polynucleotide of the present invention shall be provided, preferably, either as an isolated polynucleotide (i.e. isolated from its natural context) or in genetically modified form. The polynucleotide, preferably, is DNA including cDNA or RNA. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides.

In a further preferred embodiment, the current invention relates to a vector comprising a polynucleotide of the current invention.

The term "vector", preferably, encompasses phage, plasmid, viral or retroviral vectors as well as artificial chromosomes, such as bacterial or yeast artificial chromosomes. Moreover, the term also relates to targeting constructs which allow for random or site- directed integration of the targeting construct into genomic DNA. Such target constructs, preferably, comprise DNA of sufficient length for either homologous or heterologous recombination.

The vector encompassing the polynucleotides of the present invention, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells.

More preferably, in the vector of the invention the polynucleotide of the current invention is operatively linked to expression control sequences allowing expression in prokaryotic or eukaryotic cells or isolated fractions thereof. Expression of said polynucleotide comprises transcription of the polynucleotide, preferably into a translatable mRNA.

Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements include transcriptional as well as translational enhancers. Possible regulatory elements permitting expression in prokaryotic host cells comprise, e.g., the lac, trp or tac promoter and T7 promoter in E. coli, and examples for regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells.

More preferably, inducible expression control sequences are used in an expression vector encompassed by the present invention. Such inducible vectors comprise tet or lac operator sequences or sequences inducible by heat shock or other environmental factors. Suitable expression control sequences are well known in the art. Beside elements which are responsible for the initiation of transcription, such regulatory elements, preferably, also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (InVitrogene) or pSPORT1 (GIBCO BRL). Preferably, said vector is an expression vector and a gene transfer or targeting vector. Expression vectors derived from viruses such as bacteriophages, retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

In another preferred embodiment, the current invention relates to a host cell comprising a fusion polypeptide, a polynucleotide or a vector of the current invention.

As used herein, the term "host cell", preferably, relates to a cell capable to express the fusion polypeptide of the current invention from the polynucleotide and / or the vector of the current invention. Preferably, said host cell is a bacterial cell, more preferably a bacterial cell from the family Enterobacteriaceae, still more preferably a bacterial cell from the species Escherichia coli (E. coli), and most preferably a bacterial cell from the E. coli strains BL21(DE3) and Rosetta. It is, however, also contemplated by the current invention that the host cell is a eukaryotic cell, e.g. a yeast or mammalian, preferably human, cell.

Another preferred embodiment of the current invention is a medicament comprising the fusion protein and / or the polynucleotide and / or the vector and / or the host cell of the current invention.

The term "medicament" as used herein comprises the compounds of the present invention and optionally one or more pharmaceutically acceptable carrier. The compounds of the present invention can be formulated as pharmaceutically acceptable salts. Acceptable salts comprise acetate, methylester, HCl, sulfate, chloride and the like. The medicaments are, preferably, administered topically or systemically. Suitable routes of administration conventionally used for drug administration are subcutaneous, intramuscular, oral, intravenous, or parenteral administration as well as inhalation. However, depending on the nature and mode of action of a compound, the medicaments may be administered by other routes as well. For example, polynucleotide compounds may be administered in a gene therapy approach by using viral vectors or viruses or liposomes. Moreover, the compounds can be administered in combination with other drugs either in a common medicament or as separated medicaments wherein said separated medicaments may be provided in form of a kit of parts.

The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The diluent(s) is/are selected so as not to affect the biological activity of the combination. Examples of such diluents are distilled water, physiological saline, Ringer's solutions, dextrose solution, and Hank's solution. In addition, the medicament or formulation may also include other carriers, adjuvants, or nontoxic, nontherapeutic, nonimmunogenic stabilizers and the like.

A therapeutically effective dose refers to an amount of the compounds to be used in a medicament of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the medicament should be in the range of 1 µg to 10 mg units per day. If the regimen is a continuous infusion, it should also be in the range of 1 µg to 10 mg units per kilogram of body weight per minute, respectively. Progress can be monitored by periodic assessment. However, depending on the subject and the mode of administration, the quantity of substance administration may vary over a wide range from about 1 µg per individuum to about 10 mg per kg body mass, preferably.

The medicaments and formulations referred to herein are administered at least once in order to treat or ameliorate or prevent a disease or condition recited in this specification. However, the said medicaments may be administered more than one time, for example from one to four times daily up to a non-limited number of days.

Specific medicaments are prepared in a manner well known in the pharmaceutical art and comprise at least one active compound referred to herein above in admixture or otherwise associated with a pharmaceutically acceptable carrier or diluent. For making those specific medicaments, the active compound(s) will usually be mixed with a carrier or the diluent, or enclosed or encapsulated in a capsule, sachet, cachet, paper or other suitable containers or vehicles. The resulting formulations are to be adapted to the mode of administration, i.e. in the forms of tablets, capsules, suppositories, solutions, suspensions or the like. Dosage recommendations shall be indicated in the prescribers or users instructions in order to anticipate dose adjustments depending on the considered recipient.

The term "preventing" refers to retaining health with respect to the diseases or disorders referred to herein, preferably HPV-related disease, more preferably HPV-related cancer, for a certain period of time in a subject. Preferably, health is retained for an extended period of time with respect to said diseases or disorders. More preferably, retainment of health is enabled by a long-lasting, most preferably life-long, immune-response against HPV. It will be understood that the said period of time is dependent on the amount of the drug compound which has been administered and individual factors of the subject. It is to be understood that prevention may not be effective in all subjects treated with the compound according to the present invention. However, the term requires that a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population.

The term "treating" refers to ameliorating the diseases or disorders referred to herein above or the symptoms accompanied therewith to a significant extent. Said treating as used herein also includes an entire restoration of the health with respect to the diseases or disorders referred to herein. It is to be understood that treating as used in accordance with the present invention may not be effective in all subjects to be treated. However, the term shall require that a statistically significant portion of subjects suffering from a disease or disorder referred to herein can be successfully treated. Whether a portion is statistically significant can be determined without further ado by the person skilled in the art using various well known statistic evaluation tools discussed herein above.

In a further preferred embodiment, the current invention relates to a method for the manufacture of a medicament comprising the steps of a) providing the fusion protein and / or the polynucleotide and / or the vector and / or the host cell of the current invention and b) formulating said fusion protein and / or said polynucleotide and / or said vector and / or said host cell as a medicament for the prevention and / or treatment of HPV-related disease.

All references cited in this specification are herewith incorporated by reference with respect to their entire disclosure content and the disclosure content specifically mentioned in this specification.

The figures show:

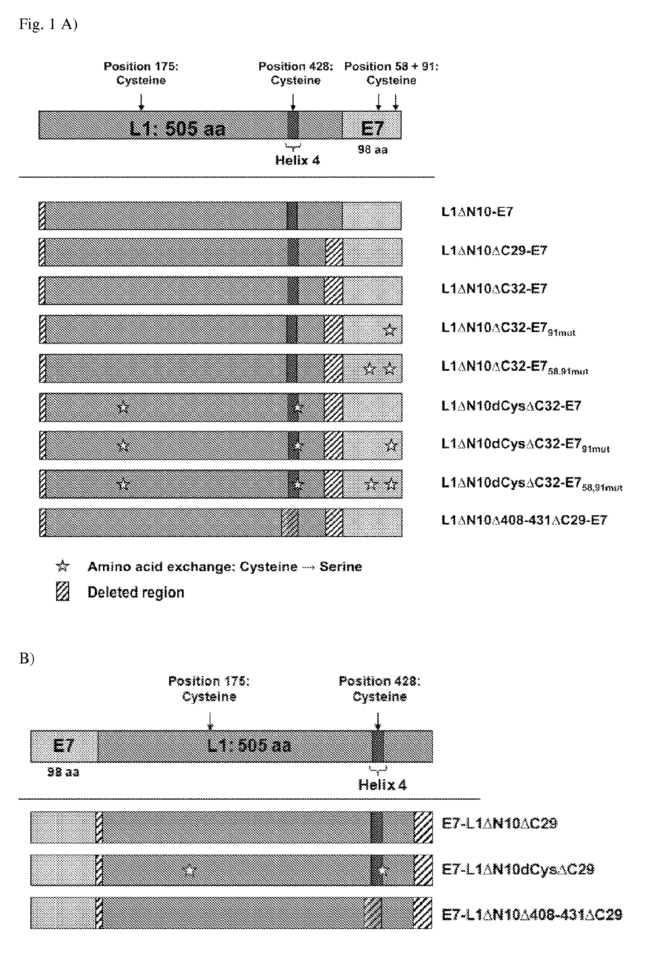

Fig. 1: Constructs for the expression of chimeric fusion proteins comprising peptides of the HPV16 E7 and L1 polypeptides in E.coli.
   The modifications/features of each construct are indicated as follows: dark grey, helix 4; hatched line, deleted region; asterisk, cysteine to serine exchange. To the right of each construct map its name. All fusion genes were cloned into the pGex 4T-3 vector to allow for their expression and purification as GST-fusion proteins and subsequent thrombin-mediated cleavage from the GST-tag. A) Different deletions and mutations were introduced into the L1E7 fusion gene in order to obtain chimeric fusion proteins with higher solubility and that are able to form more homogenous structures. The nine different constructs are depicted in cartoon format (L1: grey; E7: light grey). For comparison the original HPV16 L1E7 fusion gene is shown at the top. B) Chimeric fusion proteins carrying the E7 fused to the N-terminus of the L1 protein. In order to optimize the solubility of the chimeric fusion proteins, the E7 protein was fused to the N-terminus of the L1 protein carrying various modifications that proved to be advantageous for the L1E7 fusion proteins: the deletion of 29 C-terminal amino acids, the lack of the two cysteine residues at positions 175 and 428, and the deletion of the helix 4. The three constructs generated are depicted in cartoon format. For comparison, the unmodified E7-L1 fusion gene is shown at the top.

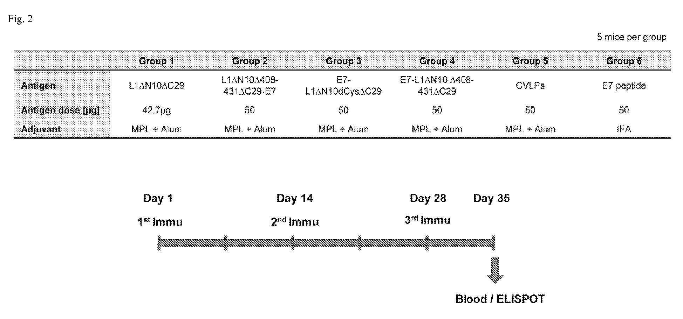

Fig. 2: Immunogenicity of the E7-L1 proteins purified from E.coli - Immunization scheme.
Fig. 3: Cellular immune response after vaccination with the E7-L1 fusion proteins.
   C57BL/6 mice (5 per group) were immunized three times s.c. at biweekly intervals with 50 µg of the E7-L1ΔN10dCysΔC29 or the E7-L1ΔN10Δ408-431ΔC29 protein purified from *E.coli*. For comparison, one group of mice was treated with 50 µg of the L1ΔN10Δ408-431ΔC29-E7 protein, while another group was injected with 50 µg of CVLPs purified from insect cells. Control mice received 42.7 µg of L1ΔN10ΔC29 capsomeres, or E7 peptide (E7₄₉₋₅₇) + IFA. All antigens (except for the E7 peptide) were administered together with 50 µg of the Sigma adjuvant system (MPL) and 500 µg of aluminium hydroxide (Alum). All antigens were treated with triton X-114 and were almost LPS free (0 to 3 EU per injection). Seven days after the last immunization, splenocytes were analysed for the presence of L1- (A) and E7-specific (B) CTLs by IFNγ ELISPOT.

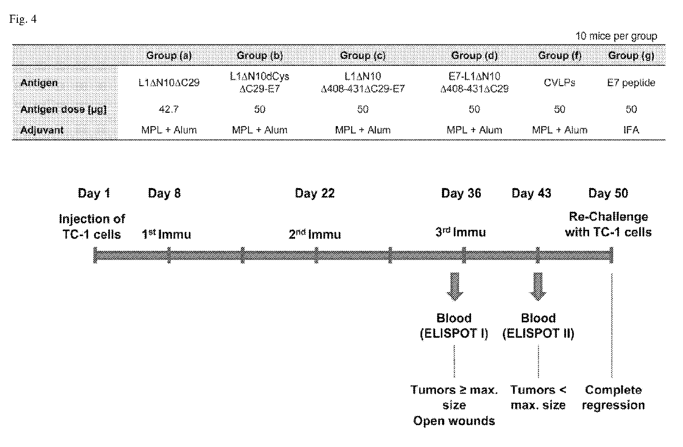

Fig. 4: Therapeutic potential of the most promising chimeric fusion protein candidates - Immunization scheme.

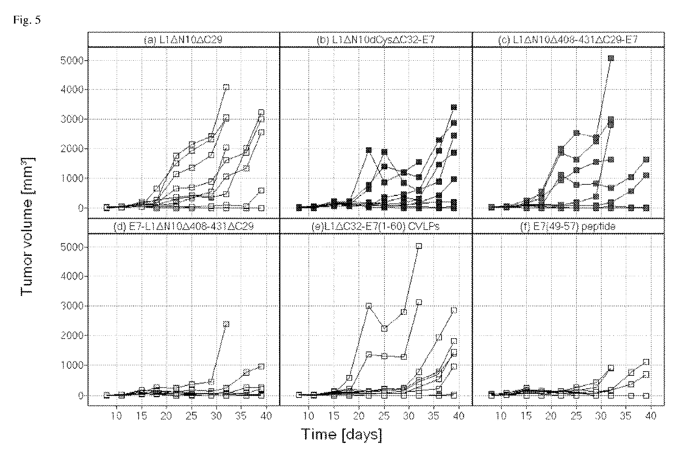

Fig. 5: Tumor growth in mice immunized with the chimeric fusion proteins.
   C57BL/6 mice were injected on day 0 with 5x10⁵ TC-1 cells and then immunized (10 mice per group) on day 8 s.c. with 50 µg of either L1ΔN10dCysΔC32-E7 (b), L1ΔN10Δ408-431ΔC29-E7 (c) or E7-L1ΔN10Δ408-431ΔC29 protein (d) (Figure 77). For comparison, one group of mice received 50 µg of CVLPs (e) purified from insect cells. Control mice were vaccinated with 42.7 µg L1ΔN10ΔC29 capsomeres (a) or 50 µg of E7 peptide (f). Except for the E7 peptide, which was co-administered with IFA, all antigens were injected in combination with Sigma Adjuvant system (MPL) and Alum. All proteins were almost LPS-free (0-6 EU per injection). Two booster immunizations were performed at biweekly intervals. Tumor volumes were measured twice per week using a caliper. On day 32, mice with tumors larger than 3 cm³, or with open wounds at the tumor site, were taken out of the experiment. The time course of the tumor growth is shown for each single mouse. Mice vaccinated with the E7-L1ΔN10Δ408-431ΔC29 protein (d) showed the strongest anti-tumor activity; whereas, for the L1E7 fusion proteins (b and c), the majority of the mice exhibited tumor growth comparable to the negative control group (a).

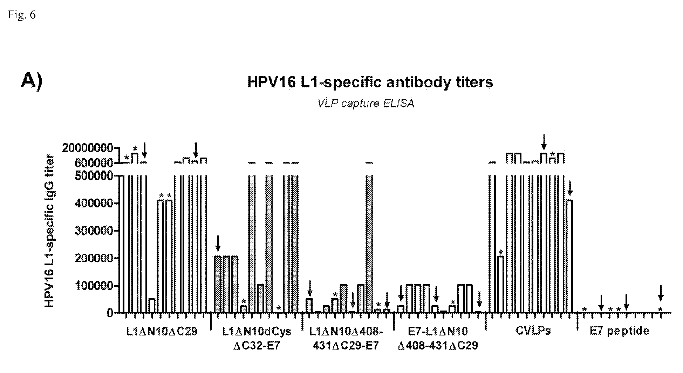

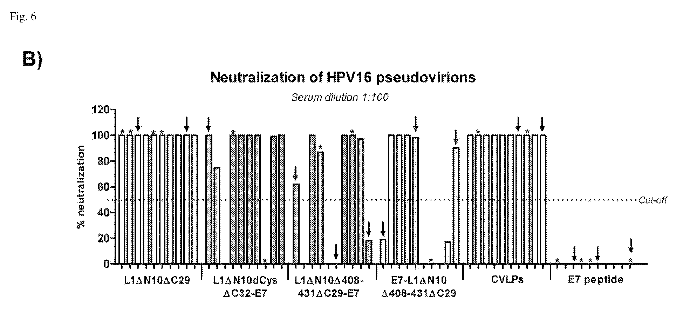

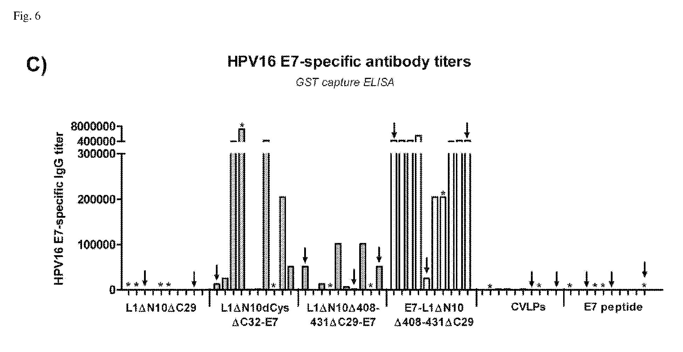

Fig 6: Humoral immune response after immunization with different chimeric fusion proteins.
   C57BL/6 mice were injected with TC-1 tumor cells on day 0 and then immunized three times s.c. at biweekly intervals (starting on day 8) with 50 µg of the L1ΔN10dCysΔC32-E7, the L1ΔN10Δ408-431ΔC29-E7 or the E7-L1ΔN10Δ408-431ΔC29 proteins. For comparison, one group of mice received 50 µg of CVLPspurified from insect cells. Control mice were vaccinated with 42.7 µg L1ΔN10ΔC29 capsomeres or 50 µg of E7 peptide. Except for the E7 peptide, which was co-administered with IFA, all antigens were injected in combination with the Sigma Adjuvant system (MPL) and Alum. All proteins were almost LPS-free (0-6 EU per injection). Seven days after the last immunization, sera were analysed for L1-specific antibody titers by VLP capture ELISA (A) and neutralization of HPV16 pseudovirions (B). E7-specific antibody titers were determined by GST capture ELISA (C). The asterisks mark sera derived from mice that had been taken out before the end of the tumor regression experiment as their tumor sizes extended the maximum volume, or because of open wounds; the values therefore correspond to 12 days after the 2^{nd} immunization. The arrows indicate sera of mice that showed complete regression.
Fig. 7 Alignment of amino acid sequences of L1 proteins of four different HPV genotypes
   The amino acid sequences of the L1 proteins of HPV16 (Genbank Ace. No ACN91182.1 GI:225423352, SEQ ID NO: 2), HPV18 (Genbank Acc. No ADC35723.1 GI:285804415, SEQ ID NO: 3), HPV33 (Genbank Acc. No AAA46964.1 GI:463183, SEQ ID NO: 4), and HPV35 (Genbank Acc. No AAA46972.1 GI:333058, SEQ ID NO: 5) were aligned using the ClustalW software. For HPV 18 the N-terminal 61 amino acids were omitted in SEQ ID NO: 3 and in the alignment as compared to Genbank Acc. No ADC35723.1, since these would create an N-terminal extension relative to the other L1 proteins. Positions of the first 10 N-terminal amino acids (N10), the cysteine residue forming a disulfide bond in an HPV capsid deleted in some of the constructs (dCys, Position 175 in the HPV16 L1), helix 4, the cysteine residue within helix 4 (position 428 in the HPV16 L1, C428) and the C-terminal 29 amino acids (C29) are indicated.

The following Examples shall merely illustrate the invention. They shall not be construed, whatsoever, to limit the scope of the invention.

### Examples

### Example 1: Biological Materials

### Bacteria:

*E.coli* Rosetta (Stratagene (La Jolla, CA, USA)); Genotype: B F'ompT hsdS(r_{B}" m_{B}") dcm⁺ Tetr gal A(DE3) endA Hte [argW ileX glyT, leuW, proL Camr]

### Plasmids:

pGex 4T-3(GE Healthcare, Uppsala, Sweden) Used for the expression of GST-fusion proteins under the control of the tac promoter (de Boer et al., 1983) in *E.coli.*

The following modification key gives an overview of the used abbreviations used to simplify the list of plasmids:
- L1:: HPV16L1 gene(wildtype)
- ΔN10:: deletion of 10 aas at the N-terminus
- ΔC29:: deletion of 29 aas at the C-terminus
- ΔC32:: deletion of 32 aas at the C-terminus
- dCys:: two point mutations C175S and C428S that replace two cysteine residues with sehne residues *(dCys - double Cys)*
- Δ408-431:: deletion of the aas 408-431 that comprise the helix 4 and a number of adjacent aas
- E7:: HPV 16E7 gene (wild type)

### Peptides

HPV16 E7 49-57: RAHYNIVTF, H2-Db-restricted CTL epitope of HPV16 E7

### Mice:

Female C57BL/6 mice at an age of 8 to 12 weeks were specified pathogen-free and maintained under pathogen-free conditions at the animal facilities of the German Cancer Research Center (DKFZ, Heidelberg, Germany).

C57BL/6 (H2D^{b}) mice were purchased from Charles River WIGA Laboratories (Sulzfeld, Germany).

Purification of chimeric fusion proteins combining HPV 16 L1 and E7 from E. coli

400 ml of TBₐₘₚ or TB_{amp/cam} medium were inoculated in a ratio of 1:100 with an overnight culture of the plasmid-containing bacteria and incubated with shaking at 200 rpm at 37°C. At an optical density (OD600nm) of 4.0, bacteria were cooled for 5 min on ice before adding IPTG to a final concentration of 0.2 mM to induce the protein expression. After 16 to 18 h incubation at 200 rpm at RT, bacteria were transferred into 50 ml-centrifuge tubes and harvested by centrifugation for 10 min at 4,000 rpm at 4°C. Bacteria pellets resulting from 100 ml of culture were resuspended in 40 ml buffer LE (50 mM Tris, 0.2 M NaCl, 2 mM DTT, pH 8.2) supplemented with a complete protease inhibitor cocktail tablet. Bacteria were lysed using a high-pressure homogeniser (French press). Lysates were cleared by centrifugation for 30 min at 20,000 rpm at 4°C (F28/50 rotor) and then loaded slowly (2 ml/min) onto a 5 ml-GSTrap column, equilibrated in buffer LE, for 16 to 24 h at 4°C. After washing the column with 10 to 20 bed volumes of buffer LE (5 ml/min), 80 u of thrombin protease in 5 ml buffer LE were added and the cleavage reaction was carried out at RT for 16 hours. Subsequently, the chimeric protein was eluted in 5 ml buffer LE. The GSTrap columns were purified by washing with 2 CVs (column volumes) of 20 mM glutathione solution and 5 CVs of cleaning solution. Afterwards columns were washed with 20-30 CVs of PBS and stored in 20% ethanol at 4°C.

For further purification the salt concentration of the GSTrap column eluate was adjusted to 0.5 M using a 5 M NaCl stock solution. The DTT concentration was raised to 5 mM and 0.01% Tween80 was added. After 15 min incubation on ice, the volume of the eluate was decreased to 1.5 ml by centrifugation at 4,000 rpm at 4°C using an Amicon Ultra-15 Centrifugal Filter Unit (100 kDa). Subsequently, the concentrated eluate was run on a Superdex200 size exclusion chromatography column in buffer LE_{mod} (Buffer LE + 3 mM DTT, 0.01 % Tween80). The presence of the chimeric fusion proteins in the peak fractions was confirmed by western blot analysis.

### GST-capture ELISA to determine E7-specific antibody titers

The GST-capture ELISA was performed using the protocol described previously (Sehr et al., 2001). 96well microtiter plates (Nunc) were coated overnight with 200 ng/well in a volume of 50 µl of glutathione casein in carbonate buffer at 4°C. Plates were washed with PBS-T and blocked with blocking solution for 1 h at 37°C. Subsequently, GST-antigen lysates were diluted to a final concentration of 0.25 µg/µl in blocking solution and loaded onto the plates (50 µl/well). After 1 h at 37°C plates were washed and the sera of immunized mice were applied in duplicates in a 2fold dilution series, starting from 1:50-1:12,800 and ending at 51,200-819,200, and were incubated for 1 h at 37°C.

Plates were washed and a horseradish peroxidase conjugated goat-anti-mouse IgG antibody (Dianova) was added (50 µl/well; 1:5,000 dilution). After 1 h at 37°C, 50 µl per well TMB (tetramethylbenzidine dihydrochloride) substrate (Sigma) were added. The reaction was carried out for 5 to 30 min and then stopped with 50 µl per well of 1 M H₂SO₄. Absorbance was measured in an ELISA reader at 450 nm. Antibody titers were calculated as the reciprocal of the highest dilution that resulted in a value above cut-off (mean values of negative control mice plus three times standard deviation). If sera were derived from mice immunized with antigens expressed in *E.coli* as GST-fusion proteins, they were blocked before loading against a lysate of BL21 bacteria expressing GST alone (empty pGex4T-3 vector) to block antibodies that might have been generated against any GST or against protein contaminations from *E.coli*. Therefore, the sera (1:50 dilution) were incubated with the bacteria lysate (5 µg/µl) in blocking solution shaking moderately for 1 h at RT.

### Generation and Neutralization of HPV16 pseudovirions

HPV16 pseudovirions consist of the HPV16 L1 and L2 proteins assembled into VLPs and incorporate a reporter plasmid. The production of pseudovirions has been developed recently (Buck *et al*., 2004; Pastrana *et al.*, 2004).

The L1-specific antibodies induced after immunization with various antigens were tested for their neutralization of HPV16 pseudovirions. For the neutralization assay, 15,000 293TT cells/well were seeded into 96well cell culture plates in DMEM medium and incubated for 1 day at 37°C and 5% CO₂. The sera of immunized mice were diluted 1:100 or 1:1,000 in medium and incubated with the HPV16 pseudoviruses for 15 min at room temperature. To determine neutralization titers, sera were applied in a 4fold dilution series from 1:100 to 1:25,600. Afterwards, the sera-pseudovirion mixtures were added to the 293TT cells in a volume of 200 µl per well and incubated for 5 days at 37°C and 5% CO₂. As controls, 293TT cells were infected with pseudovirions alone (negative control) or with pseudovirions that were mixed with the HPV 16 L1-specific neutralizing monoclonal antibody Ritti01 (Rizk et al., 2008) (positive control). Subsequently, the supernatant of the cells was collected and analysed for the presence of SEAP using the chemiluminescence SEAP reporter gene assay following the manufacturer's instructions (Roche). Neutralizing activity was expressed in percent neutralization comparing to the positive control, which was set to 100%. All sera that showed neutralization above an arbitrary cut-off of 50% were regarded as neutralization positive.

### Detection of antigen-specific cytotoxic T-lymphocytes by IFNγ-ELISPOT

*Ex vivo* ELISPOT analysis was performed 7 days after the last vaccination. All steps on day 1 and 2 were carried out under sterile conditions in a flow hood. 96well MultiScreen-HTS^{™} ELISPOT plates (Millipore, Germany) were incubated for 5 min with 70 µl of 70% EtOH, washed 4times with PBS and then coated with 100 µl/well of anti-mouse IFNγ capture antibody (0.6 µg per well,) in PBS at 4°C overnight. Plates were washed 4times with PBS and then blocked with 150 µl per well of RPMI medium supplemented with 10% FCS and penicillin and streptomycin for 2 h at 37°C. Splenocytes of immunized mice were seeded into three wells in a 2fold dilution series from 1-2x10⁶ cells to 1.25-2.5x10⁵ cells per well in a volume of 100 µl. As negative control, 50 µl per well medium were added to cells of one dilution series. As positive control one row of cells was stimulated unspecifically with 1 µg per well POKEWEED mitogen. The third row received 10 µM of HPV16 L1 ₁₆₅₋₁₇₃ or the HPV16 E7₄₉₋₅₇ peptide (5.2.4.) in a volume of 50 µl. After 16 to 18 h incubation at 37°C, the cells were removed by washing 4times with PBS-0.01% Tween20 and once with PBS. Then, 0.1 µg per well of a biotinylated rat-anti-66 mouse IFNγ antibody was added for 2 h at room temperature. Unbound detection antibody was removed by washing 6times with PBS-0.01% Tween20 and once with PBS. Subsequently, 100 µl per well streptavidin-alkaline phosphatase diluted 1:1,000 in PBS were added and incubated for 30 min at room temperature in the dark. Plates were washed 3times with PBS-0.01% Tween20 and 3 times with PBS and stained for up to 10 min with 100 µl per well of 5-bromo-4-chloro-3-indolylphosphate (BCIP)/Nitro Blue Tetrozolium liquid substrate system. The reaction was stopped by rinsing the plates extensively with cold water and plates were dried overnight. Spots were quantified in an ELISPOT reader. For the IFNγ ELISPOT after the 1^{st} restimulation, a dilution series of splenocytes was performed using 2-4x10⁵ cells/well to 2.5-5x10⁴ cells/well. After 2^{nd} restimulation a dilution series of 50,000 cells/well to 6,250 cells/well was used.

### Example 2: Yield of fusion proteins comprising E7 and L1 peptides

The purification of the HPV16 L1E7 fusion proteins from *E.coli* is possible although the fusion proteins are mostly insoluble (Fig. 1A, Table 1A). In contrast, when the E7 protein was fused N-terminally of the L1 protein carrying various modifications that have been proven advantageous for the L1E7 protein - a C-terminal truncation of 29 amino acids, the replacement of the two cysteine residues at position 175 and 428 for serine residues, and the deletion of the helix (Fig 1A and B), the solubility and the yield of the fusion protein was found to be drastically increased (Table 1B):
The three constructs were transformed into *E.coli* Rosetta bacteria and their encoded proteins were expressed and purified following the protocol for the chimeric proteins. The proteins were subjected to gel filtration chromatography and subsequently analysed on a Coomassie-stained protein gel and by western blotting.
The protein concentrations were determined densitometrically and the protein yields per liter bacteria culture are shown in Table 1B. Compared to their C-terminally fused analogues (Table 1A), the protein yields were increased about 2.3fold, in case of the E7-L1ΔN10ΔC29, 3.4fold for the E7-L1ΔN10dCysΔC29, and even 4.6fold for the E7-L1ΔN10Δ408-431ΔC29 protein. The proteins were of the expected molecular weight, and the presence of L1 and E7 was confirmed by western blotting.

**Table 1 A) Yield of the differently modified L1E7 proteins purified from E.coli.**

| **Fusion protein** | **Yield per liter bacteria culture [mg]** |
|---|---|
| L1ΔN10-E7 | 0.2 |
| L1ΔN10ΔC29-E7 | 0.8 |
| L1ΔN10ΔC32-E7 | 0.5 |
| L1ΔN10ΔC32-E7₉₁ₘᵤₜ | 0.8 |
| L1ΔN10ΔC32-E7_{56.91mut} | 0.7 |
| L1ΔN10dCysΔC32-E7 | 1.3 |
| L1ΔN10dCysΔC32-E7₉₁ₘᵤₜ | 0.8 |
| L1ΔN10dCysΔC32-E7_{56.91mut} | 0.7 |
| L1ΔN 10Δ408-431ΔC29-E7 | 1.3 |

The various L1E7 fusion proteins (Fig. 1A) were purified using the optimized protocol. Protein yields were determined on a Coomassie-stained protein gel by comparison to a BSA standard. Values correspond to purifications from 1 L of bacterial culture. The highest protein yields were obtained for the L1ΔN10dCysΔC32-E7 and the L1ΔN10Δ408-431ΔC32-E7 proteins.

**Table 1 B) Protein yields for the differently modified E7-L1 proteins purified from E.coli.**

| **Fusion protein** | **Yield per liter bacteria culture [mg]** |
|---|---|
| E7-L1ΔN10ΔC26 | 1.8 |
| E7-L1ΔN10dCysΔC29 | 4.4 |
| E7-L1ΔN10Δ408-431ΔC29 | 6.0 |

The various E7-L1 proteins (Figure 1B) were expressed in and purified from E.coli using the optimized protocol for the chimeric proteins. After gel filtration, the proteins were analysed on a Coomassie-stained protein gel and protein concentrations were determined densitometrically. The protein yields are given in mg per liter bacterial culture.

### Example 3: Immunogenicity of fusion proteins: cellular immune response

The immunogenicity of different fusion proteins was compared amoung the most promising candidates in mice.

C57BL/6 mice (5 per group) were vaccinated three times s.c. at biweekly intervals with 50 µg of the E7-L1ΔN10dCysΔC29 or the E7-L1ΔN10Δ408-431ΔC29 protein. For comparison, one group of mice received 50 µg of the L1ΔN10Δ408-431ΔC29-E7 protein, and another group was injected with CVLPs (L1ΔC32-E7₁₋₆₀) purified from insect cells. Control mice were treated with either 42.7 µg of L1ΔN10ΔC29 capsomeres, corresponding to the amount of L1 injected with the E7-L1 proteins, or with 50 µg of the E7 CTL epitope-derived peptide (E7₄₉₋₅₇). Apart from the peptide, which was administered with IFA as an adjuvant, all other antigens were injected in combination with the Sigma Adjuvant system (Sigma) - which contains 50 µg of monophosphoryl lipid A (MPL) derived from *Salmonella enterica* serovar Minnesota R595, and 50 µg of synthetic trehalose dicorynomycolate in squalene and Tween 80 - and 500 µg of aluminium hydroxide (Alum). This adjuvant mix is most similar to the Cervarix^{®} adjuvant AS04, which is not commercially available. All antigens were almost endotoxin-free, as they were treated with triton X-114 (see chapter 7.3.; for protocol see 6.6.2.), resulting in LPS amounts of 0 to 3 EU per injection.

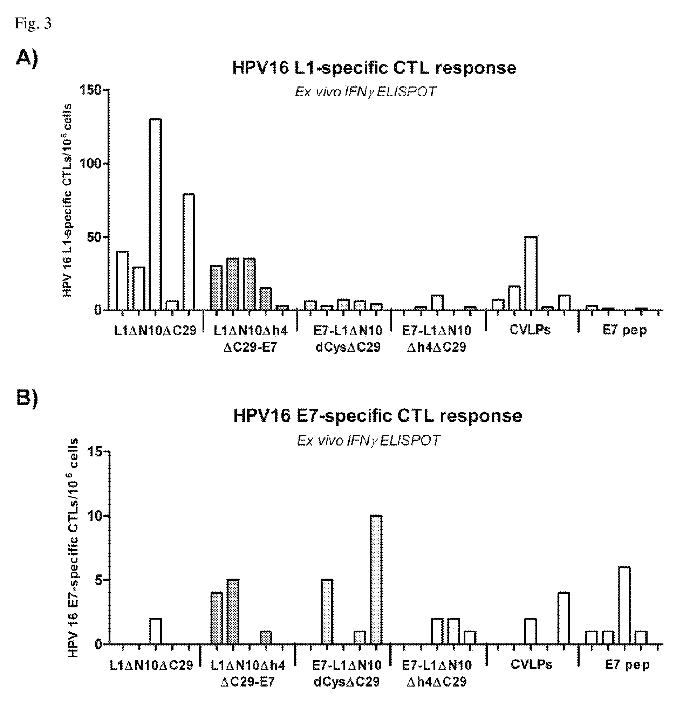

Seven days after the last immunization, the cellular immune response was analysed by the detection of L1- and E7-specific cytotoxic T-lymphocytes in the splenocytes of the immunized mice using IFNγ ELISPOT (Fig. 3).

For the mice that received the L1ΔN10ΔC29 capsomeres and the L1ΔN10Δ408-431ΔC29-E7 fusion protein, similar numbers of L1-specific CTLs were detected (median spot number of 40 and 30). Immunization with the E7-L1 fusion proteins resulted in lower numbers, or even undetectable CTLs. Also, after injection of the CVLPs, very low spot numbers were found. The E7-specific CTL response was very low for all mice. Even for the positive control mice immunized with the E7 peptide, only few E7-specific CTLs were detected. Though the E7-L1 proteins less efficiently induced the generation of L1-specific antibodies, immunization with the E7-L1 proteins also elicited E7-specific antibodies and low numbers of E7-specific cytotoxic T-lymphocytes.

### Example 4: Immunogenicity of fusion proteins: antitumor potential

Results presented in example 3 demonstrated that the sensitivity of detection of an E7-specific cellular immune response was rather low. This limitation notwithstanding, the therapeutic potential of the chimeric fusion proteins was investigated *in vivo* in a tumor regression assay since the aim was to develop a therapeutic vaccine - also featuring prophylactic properties - and therefore data on the *in vivo* activity are more informative.

For this experiment, TC-1 cells, which express only the HPV16 E6 and E7 proteins, were used (Lin et al., 1996) in order to exclude the possible involvement of induced L1-specific CTLs in tumor regression. Beforehand, the tumor take rate and the growth rate were analysed in relation to the number of injected TC-1 cells. The injection of 5x10⁵ tumor cells was determined to be most suitable and led to measureable tumors in 2/3 of the mice after seven days. As 60 tumor-carrying mice were necessary for the experiment (six groups of 10 mice), 90 mice had to be injected with 5x10⁵ TC-1 cells. Seven days later, the mice were examined for the appearance of tumors and were separated into six groups. On day eight, mice were immunized with one of the three most immunogenic chimeric fusion proteins determined in the experiments discussed in the previous sections: L1ΔN10dCysΔ32-E7, L1ΔN10Δ408-431ΔC29-E7 and E7-L1ΔN10Δ408-431ΔC29 proteins. The mice were vaccinated three times subcutaneously at biweekly intervals with 50 µg of one of these proteins (Fig. 4). For comparison, one group of mice received 50 µg of L1ΔC32-E7₁₋₆₀ CVLPs purified from baculovirus infected insect cells. Control mice were treated either with 42.7 µg of L1ΔN10ΔC29 capsomeres or with 50 µg of E7 peptide (E7₄₉₋₅₇). All antigens - except for the E7 peptide, which was injected in combination with IFA - were administered with Sigma Adjuvant system (Sigma) - which contains 50 µg of monophosphoryl lipid A (MPL) derived from *Salmonella enterica serovar* Minnesota R595, 50 µg of synthetic trehalose dicorynomycolate in squalene and Tween 80 - and 500 µg of aluminium hydroxide (Alum). The antigens were almost LPS-free due to several rounds of triton X-114 treatment that substantially reduced the LPS contaminations (between undetectable and 6 EU per injection). Tumor volumes were measured twice per week. Mice were taken out of the experiment when the tumor size reached the maximal volume of 3 cm³, or when open wounds appeared at the tumor site. Mice that showed complete tumor regression were re-challenged with TC-1 cells on day 50.

The time course of tumor development is depicted in Fig. 5. In contrast to mice vaccinated with L1ΔN10ΔC29 protein (a), for which 8/10 mice exhibited constant tumor growth, the tumors of the mice immunized with the L1ΔN10dCysΔC32-E7 (b), the L1ΔN10Δ408-431ΔC29-E7 (c), and the CVLPs (e), either decreased in size, neither increased or decreased, or increased slowly in response to the first immunization. However, following the second immunization (day 22), most of the mice showed growing tumors: 7/10 for the L1ΔN10dCysΔC32-E7, 6/10 for the L1ΔN10Δ408-431ΔC29-E7, and 8/10 for the CVLPs. For mice treated with the E7 peptide (e), no tumor growth was observed until day 29, though later on, 5/10 mice showed growing tumors. Treatment with the E7-L1ΔN10Δ408-431ΔC29 protein (d) resulted in regression, no growth, or highly attenuated growth compared to the group injected with the L1ΔN10ΔC29 capsomeres or the L1E7 fusion proteins. Although 4/10 mice showed tumor growth, the tumor sizes were much lower at the end of the experiment.

All 15 mice that were tumor-free at day 43 were re-challenged on day 50 with 5x10⁵ TC-1 cells. After 14 days, only the mice vaccinated with the E7 peptide developed a tumor. This can be explained by the failure of these mice to generate a T-cell memory, as the E7 peptide used for immunization (E7₄₉₋₅₇) is an H2-Db-restricted CTL epitope (Feltkamp et al., 1993).

### Example 5: Immunogenicity of fusion proteins: humoral immune response

The humoral immune response was analysed seven days after the last immunization (Fig. 4, day 43) - or in case of the mice that were taken out of the experiment early due to too large tumors or open wounds, 12 days after the second immunization (day 34). Blood samples were taken and sera were analysed for L1-specific antibody titers by VLP capture ELISA, and for neutralizing activity against HPV16 pseudovirions (Fig. 6).

Comparing the L1-specific antibody titers after immunization with the various chimeric fusion proteins (Fig. 6A), injection of the L1ΔN10dCysΔC32-E7 protein (median titer of 204,800) induced statistically significantly higher titers than the L1ΔN10Δ408-431ΔC29-E7 (median titer of 38,400; *p=0.041*) and the E7-L1ΔN10Δ408-431ΔC29 proteins (median titer of 64,000; *p=0.021*). There was no statistically significant difference between the group that received the L1ΔN10Δ408-431ΔC29-E7 protein and the group treated with the E7-L1ΔN10Δ408-431ΔC29 protein. For the CVLP group, the L1-specific antibody titers were similar to those detected in the LΔ1N10C29 group.

Neutralization of HPV16 pseudovirions in a serum dilution of 1:100 was shown for 8/10 mice immunized with the L1ΔN10dCysΔC32-E7, 6/10 mice treated with the L1ΔN10Δ408-431ΔC29-E7, and 5/10 mice receiving the E7-L1ΔN10Δ408-431ΔC29 protein (Fig. 6B).

In the sera of mice vaccinated with the E7-L1ΔN10Δ408-431ΔC29 protein (median titer of 819,200), statistically significantly higher E7-specific antibody titers were detected as compared to the sera of mice immunized with the L10N10dCysΔC32-E7 (median titer of 38,200; *p=0.045)* or the L1ΔN10Δ408-431ΔC29-E7 (median titer of 9,600; *p*<*0.001*)(Fig. 6C). The injection of CVLPs elicited no or very low E7-specific antibody responses.

### Example 6: alignment of HPV L1 amino acid sequences

Sequences of HPV16, 18, 33, and 35 were aligned using the Clustal W multiple sequence alignment program (Larkin et al. (2007) "Clustal W and Clustal X version 2.0" Bioinformatics, Volume: 23 (Issue: 21), 2947-2948).

## Claims

1. A fusion polypeptide comprising a) a first domain comprising a HPV E7 polypeptide and b) a second domain comprising at least one peptide from a second polypeptide, wherein the first domain is located N-terminally of the second domain.

2. The fusion polypeptide of claim 1, wherein the first domain comprises the HPV16 E7 polypeptide.

3. The fusion polypeptide of claims 1 or 2, wherein the second polypeptide is an HPV L1 polypeptide.

4. The fusion polypeptide of any one of claims 1 to 3, wherein the second domain is an HPV L1 polypeptide.

5. The fusion polypeptide of any one of claims 1 to 4, wherein the second domain is an HPV16 L1 polypeptide.

6. The fusion polypeptide of any one of claims 1 to 5, wherein the second domain is an HPV L1 polypeptide comprising a deletion of the first 10 N-terminal amino acids or a deletion of the first 10 N-terminal amino acids and 29-32 amino acids of the C-Terminus and / or a deletion of helix 4 and / or at least one mutation of a cysteine residue forming a disulfide bond in a HPV capsid.

7. The fusion polypeptide of any one of claims 1 to 6, wherein the second domain is an HPV16 L1 polypeptide comprising a deletion of the first 10 N-terminal amino acids / a deletion of the first 10 N-terminal amino acids and 29-32 amino acids of the C-Terminus and / or a deletion of helix 4 and / or at least one mutation of a cysteine residue forming a disulfide bond in a HPV capsid.

8. A polynucleotide comprising a sequence encoding the fusion polypeptide of any one of claims 1 to 7.

9. A vector comprising the polynucleotide of claim 8.

10. A host cell comprising the fusion polypeptide of any one of claims 1 to 7 and / or the polynucleotide of claim 8 and / or the vector of claim 9.

11. A medicament comprising the fusion protein of any one of claims 1 to 7 and / or the polynucleotide of claim 8 and / or the vector of claim 9 and / or the host cell of claim 10.

12. The fusion protein of any one of claims 1 to 7 and / or the polynucleotide of claim 8 and / or the vector of claim 9 and / or the host cell of claim 10 as a medicament for the prevention and / or treatment of HPV-related disease.

13. The fusion protein of any one of claims 1 to 7 and / or the polynucleotide of claim 8 and / or the vector of claim 9 and / or the host cell of claim 10 as the medicament of claim 12, wherein the HPV-related disease is HPV-related cancer.

14. A method for the manufacture of a medicament comprising the steps of a) providing the fusion protein of any one of claims 1 to 7 and / or the polynucleotide of claim 8 and / or the vector of claim 9 and / or the host cell of claim 10 and b) formulating said fusion protein and / or said polynucleotide and / or said vector and / or said host cell as a medicament for the prevention and / or treatment of HPV-related disease.

15. The method of claim 13, wherein the HPV-related disease is HPV-related cancer.


SEQUENCE LISTING

<110> Deutsches Krebsforschungszentrum

<120> N-terminal HPV E7 fusion proteins

<130> DK66719EP

<160> 5

<170> PatentIn version 3.3

<210> 1
<211> 98
<212> PRT
<213> Human papillomavirus type 16

<400> 1

Met His Gly Asp Thr Pro Thr Leu His Glu Tyr Met Leu Asp Leu Gln
1               5                   10                  15

Pro Glu Thr Thr Asp Leu Tyr Cys Tyr Glu Gln Leu Ser Asp Ser Ser
            20                  25                  30

Glu Glu Glu Asp Glu Ile Asp Gly Pro Ala Gly Gln Ala Glu Pro Asp
        35                  40                  45

Arg Ala His Tyr Asn Ile Val Thr Phe Cys Cys Lys Cys Asp Ser Thr
    50                  55                  60

Leu Arg Leu Cys Val Gln Ser Thr His Val Asp Ile Arg Thr Leu Glu
65                  70                  75                  80

Asp Leu Leu Met Gly Thr Leu Gly Ile Val Cys Pro Ile Cys Ser Gln
                85                  90                  95

Lys Pro

<210> 2
<211> 503
<212> PRT
<213> Human papillomavirus type 16

<400> 2

Met Ser Leu Trp Leu Pro Ser Glu Ala Thr Val Tyr Leu Pro Pro Val
1               5                   10                  15

Pro Val Ser Lys Val Val Ser Thr Asp Glu Tyr Val Ala Arg Thr Asn
            20                  25                  30

Ile Tyr Tyr His Ala Gly Thr Ser Arg Leu Leu Ala Val Gly His Pro
        35                  40                  45

Tyr Phe Pro Ile Lys Lys Pro Asn Asn Asn Lys Ile Leu Val Pro Lys
    50                  55                  60

Val Ser Gly Leu Gln Tyr Arg Val Phe Arg Ile His Leu Pro Asp Pro
65                  70                  75                  80

Asn Lys Phe Gly Phe Pro Asp Thr Ser Phe Tyr Asn Pro Asp Thr Gln
                85                  90                  95

Arg Leu Val Trp Ala Cys Val Gly Val Glu Val Gly Arg Gly Gln Pro
            100                 105                 110

Leu Gly Val Gly Ile Ser Gly His Pro Leu Leu Asn Lys Leu Asp Asp
        115                 120                 125

Thr Glu Asn Ala Ser Ala Tyr Ala Ala Asn Ala Gly Val Asp Asn Arg
    130                 135                 140

Glu Cys Ile Ser Met Asp Tyr Lys Gln Thr Gln Leu Cys Leu Ile Gly
145                 150                 155                 160

Cys Lys Pro Pro Ile Gly Glu His Trp Gly Lys Gly Ser Pro Cys Thr
                165                 170                 175

Asn Val Ala Val Asn Pro Gly Asp Cys Pro Pro Leu Glu Leu Ile Asn
            180                 185                 190

Thr Val Ile Gln Asp Gly Asp Met Val Asp Thr Gly Phe Gly Ala Met
        195                 200                 205

Asp Phe Thr Thr Leu Gln Ala Asn Lys Ser Glu Val Pro Leu Asp Ile
    210                 215                 220

Cys Thr Ser Ile Cys Lys Tyr Pro Asp Tyr Ile Lys Met Val Ser Glu
225                 230                 235                 240

Pro Tyr Gly Asp Ser Leu Phe Phe Tyr Leu Arg Arg Glu Gln Met Phe
                245                 250                 255

Val Arg His Leu Phe Asn Arg Ala Gly Ala Val Gly Glu Asn Val Pro
            260                 265                 270

Asp Asp Leu Tyr Ile Lys Gly Ser Gly Ser Thr Ala Asn Leu Ala Ser
        275                 280                 285

Ser Asn Tyr Phe Pro Thr Pro Ser Gly Ser Met Val Thr Ser Asp Ala
    290                 295                 300

Gln Ile Phe Asn Lys Pro Tyr Trp Leu Gln Arg Ala Gln Gly His Asn
305                 310                 315                 320

Asn Gly Ile Cys Trp Gly Asn Gln Leu Phe Val Thr Val Val Asp Thr
                325                 330                 335

Thr Arg Ser Thr Asn Met Ser Leu Cys Ala Ala Ile Ser Thr Ser Glu
            340                 345                 350

Thr Thr Tyr Lys Asn Thr Asn Phe Lys Glu Tyr Leu Arg His Gly Glu
        355                 360                 365

Glu Tyr Asp Leu Gln Phe Ile Phe Gln Leu Cys Lys Ile Thr Leu Thr
    370                 375                 380

Ala Asp Val Met Thr Tyr Ile His Ser Met Asn Ser Thr Ile Leu Glu
385                 390                 395                 400

Asp Trp Asn Phe Gly Leu Gln Pro Pro Pro Gly Gly Thr Leu Glu Asp
                405                 410                 415

Thr Tyr Arg Phe Val Thr Ser Gln Ala Ile Ala Cys Gln Lys His Thr
            420                 425                 430

Pro Pro Ala Pro Lys Glu Asp Pro Leu Lys Lys Tyr Thr Phe Trp Glu
        435                 440                 445

Val Asn Leu Lys Glu Lys Phe Ser Ala Asp Leu Asp Gln Phe Pro Leu
    450                 455                 460

Gly Arg Lys Phe Leu Leu Gln Ala Gly Leu Lys Ala Lys Pro Lys Phe
465                 470                 475                 480

Thr Leu Gly Lys Arg Lys Ala Thr Pro Thr Thr Ser Ser Thr Ser Thr
                485                 490                 495

Thr Ala Lys Arg Lys Lys Arg
            500

<210> 3
<211> 507
<212> PRT
<213> Human papillomavirus type 18

<400> 3

Met Ala Leu Trp Arg Pro Ser Asp Asn Thr Val Tyr Leu Pro Pro Pro
1               5                   10                  15

Ser Val Ala Arg Val Val Asn Thr Asp Asp Tyr Val Thr Arg Thr Ser
            20                  25                  30

Ile Phe Tyr His Ala Gly Ser Ser Arg Leu Leu Thr Val Gly Asn Pro
        35                  40                  45

Tyr Phe Arg Val Pro Ala Gly Gly Gly Asn Lys Gln Asp Ile Pro Lys
    50                  55                  60

Val Ser Ala Tyr Gln Tyr Arg Val Phe Arg Val Gln Leu Pro Asp Pro
65                  70                  75                  80

Asn Lys Phe Gly Leu Pro Asp Thr Ser Ile Tyr Asn Pro Glu Thr Gln
                85                  90                  95

Arg Leu Val Trp Ala Cys Ala Gly Val Glu Ile Gly Arg Gly Gln Pro
            100                 105                 110

Leu Gly Val Gly Leu Ser Gly His Pro Phe Tyr Asn Lys Leu Asp Asp
        115                 120                 125

Thr Glu Ser Ser His Ala Ala Thr Ser Asn Val Ser Glu Asp Val Arg
    130                 135                 140

Asp Asn Val Ser Val Asp Tyr Lys Gln Thr Gln Leu Cys Ile Leu Gly
145                 150                 155                 160

Cys Ala Pro Ala Ile Gly Glu His Trp Ala Lys Gly Thr Ala Cys Lys
                165                 170                 175

Ser Arg Pro Leu Ser Gln Gly Asp Cys Pro Pro Leu Glu Leu Lys Asn
            180                 185                 190

Thr Val Leu Glu Asp Gly Asp Met Val Asp Thr Gly Tyr Gly Ala Met
        195                 200                 205

Asp Phe Ser Thr Leu Gln Asp Thr Lys Cys Glu Val Pro Leu Asp Ile
    210                 215                 220

Cys Gln Ser Ile Cys Lys Tyr Pro Asp Tyr Leu Gln Met Ser Ala Asp
225                 230                 235                 240

Pro Tyr Gly Asp Ser Met Phe Phe Cys Leu Arg Arg Glu Gln Leu Phe
                245                 250                 255

Ala Arg His Phe Trp Asn Arg Ala Gly Thr Met Gly Asp Thr Val Pro
            260                 265                 270

Gln Ser Leu Tyr Ile Lys Gly Thr Gly Met Arg Ala Ser Pro Gly Ser
        275                 280                 285

Cys Val Tyr Ser Pro Ser Pro Ser Gly Ser Ile Val Thr Ser Asp Ser
    290                 295                 300

Gln Leu Phe Asn Lys Pro Tyr Trp Leu His Lys Ala Gln Gly His Asn
305                 310                 315                 320

Asn Gly Val Cys Trp His Asn Gln Leu Phe Val Thr Val Val Asp Thr
                325                 330                 335

Thr Arg Ser Thr Asn Leu Thr Ile Cys Ala Ser Thr Gln Ser Pro Val
            340                 345                 350

Pro Gly Gln Tyr Asp Ala Thr Lys Phe Lys Gln Tyr Ser Arg His Val
        355                 360                 365

Glu Glu Tyr Asp Leu Gln Phe Ile Phe Gln Leu Cys Thr Ile Thr Leu
    370                 375                 380

Thr Ala Asp Val Met Ser Tyr Ile His Ser Met Asn Ser Ser Ile Leu
385                 390                 395                 400

Glu Asp Trp Asn Phe Gly Val Pro Pro Pro Pro Thr Thr Ser Leu Val
                405                 410                 415

Asp Thr Tyr Arg Phe Val Gln Ser Val Ala Ile Thr Cys Gln Lys Asp
            420                 425                 430

Ala Ala Pro Ala Glu Asn Lys Asp Pro Tyr Asp Lys Leu Lys Phe Trp
        435                 440                 445

Asn Val Asp Leu Lys Glu Lys Phe Ser Leu Asp Leu Asp Gln Tyr Pro
    450                 455                 460

Leu Gly Arg Lys Phe Leu Val Gln Ala Gly Leu Arg Arg Lys Pro Thr
465                 470                 475                 480

Ile Gly Pro Arg Lys Arg Ser Ala Pro Ser Ala Thr Thr Ser Ser Lys
                485                 490                 495

Pro Ala Lys Arg Val Arg Val Arg Ala Arg Lys
            500                 505

<210> 4
<211> 499
<212> PRT
<213> Human papillomavirus type 33

<400> 4

Met Ser Val Trp Arg Pro Ser Glu Ala Thr Val Tyr Leu Pro Pro Val
1               5                   10                  15

Pro Val Ser Lys Val Val Ser Thr Asp Glu Tyr Val Ser Arg Thr Ser
            20                  25                  30

Ile Tyr Tyr Tyr Ala Gly Ser Ser Arg Leu Leu Ala Val Gly His Pro
        35                  40                  45

Tyr Phe Ser Ile Lys Asn Pro Thr Asn Ala Lys Lys Leu Leu Val Pro
    50                  55                  60

Lys Val Ser Gly Leu Gln Tyr Arg Val Phe Arg Val Arg Leu Pro Asp
65                  70                  75                  80

Pro Asn Lys Phe Gly Phe Pro Asp Thr Ser Phe Tyr Asn Pro Asp Thr
                85                  90                  95

Gln Arg Leu Val Trp Ala Cys Val Gly Leu Glu Ile Gly Arg Gly Gln
            100                 105                 110

Pro Leu Gly Val Gly Ile Ser Gly His Pro Leu Leu Asn Lys Phe Asp
        115                 120                 125

Asp Thr Glu Thr Gly Asn Lys Tyr Pro Gly Gln Pro Gly Ala Asp Asn
    130                 135                 140

Arg Glu Cys Leu Ser Met Asp Tyr Lys Gln Thr Gln Leu Cys Leu Leu
145                 150                 155                 160

Gly Cys Lys Pro Pro Thr Gly Glu His Trp Gly Lys Gly Val Ala Cys
                165                 170                 175

Thr Asn Ala Ala Pro Ala Asn Asp Cys Pro Pro Leu Glu Leu Ile Asn
            180                 185                 190

Thr Ile Ile Glu Asp Gly Asp Met Val Asp Thr Gly Phe Gly Cys Met
        195                 200                 205

Asp Phe Lys Thr Leu Gln Ala Asn Lys Ser Asp Val Pro Ile Asp Ile
    210                 215                 220

Cys Gly Ser Thr Cys Lys Tyr Pro Asp Tyr Leu Lys Met Thr Ser Glu
225                 230                 235                 240

Pro Tyr Gly Asp Ser Leu Phe Phe Phe Leu Arg Arg Glu Gln Met Phe
                245                 250                 255

Val Arg His Phe Phe Asn Arg Ala Gly Thr Leu Gly Glu Ala Val Pro
            260                 265                 270

Asp Asp Leu Tyr Ile Lys Gly Ser Gly Thr Thr Ala Ser Ile Gln Ser
        275                 280                 285

Ser Ala Phe Phe Pro Thr Pro Ser Gly Ser Met Val Thr Ser Glu Ser
    290                 295                 300

Gln Leu Phe Asn Lys Pro Tyr Trp Leu Gln Arg Ala Gln Gly His Asn
305                 310                 315                 320

Asn Gly Ile Cys Trp Gly Asn Gln Val Phe Val Thr Val Val Asp Thr
                325                 330                 335

Thr Arg Ser Thr Asn Met Thr Leu Cys Thr Gln Val Thr Ser Asp Ser
            340                 345                 350

Thr Tyr Lys Asn Glu Asn Phe Lys Glu Tyr Ile Arg His Val Glu Glu
        355                 360                 365

Tyr Asp Leu Gln Phe Val Phe Gln Leu Cys Lys Val Thr Leu Thr Ala
    370                 375                 380

Glu Val Met Thr Tyr Ile His Ala Met Asn Pro Asp Ile Leu Glu Asp
385                 390                 395                 400

Trp Gln Phe Gly Leu Thr Pro Pro Pro Ser Ala Ser Leu Gln Asp Thr
                405                 410                 415

Tyr Arg Phe Val Thr Ser Gln Ala Ile Thr Cys Gln Lys Thr Val Pro
            420                 425                 430

Pro Lys Glu Lys Glu Asp Pro Leu Gly Lys Tyr Thr Phe Trp Glu Val
        435                 440                 445

Asp Leu Lys Glu Lys Phe Ser Ala Asp Leu Asp Gln Phe Pro Leu Gly
    450                 455                 460

Arg Lys Phe Leu Leu Gln Ala Gly Leu Lys Ala Lys Pro Lys Leu Lys
465                 470                 475                 480

Arg Ala Ala Pro Thr Ser Thr Arg Thr Ser Ser Ala Lys Arg Lys Lys
                485                 490                 495

Val Lys Lys

<210> 5
<211> 505
<212> PRT
<213> Human papillomavirus type 35

<400> 5

Met Ser Leu Trp Arg Ser Asn Glu Ala Thr Val Tyr Leu Pro Pro Val
1               5                   10                  15

Ser Val Ser Lys Val Val Ser Thr Asp Glu Tyr Val Thr Arg Thr Asn
            20                  25                  30

Ile Tyr Tyr His Ala Gly Ser Ser Arg Leu Leu Ala Val Gly His Pro
        35                  40                  45

Tyr Tyr Ala Ile Lys Lys Gln Asp Ser Asn Lys Ile Ala Val Pro Lys
    50                  55                  60

Val Ser Gly Leu Gln Tyr Arg Val Phe Arg Val Lys Leu Pro Asp Pro

65                    70                    75                    80

Asn Lys Phe Gly Phe Pro Asp Thr Ser Phe Tyr Asp Pro Cys Leu Gln
                85                  90                  95

Arg Leu Val Trp Ala Cys Thr Gly Val Glu Val Gly Arg Gly Gln Pro
            100                 105                 110

Leu Gly Val Gly Ile Ser Gly His Pro Leu Leu Asn Lys Leu Asp Asp
        115                 120                 125

Thr Glu Asn Leu Asn Lys Tyr Val Gly Asn Ser Gly Asn Ser Gly Thr
    130                 135                 140

Asp Asn Arg Glu Cys Ile Ser Met Asp Tyr Lys Gln Thr Gln Leu Cys
145                 150                 155                 160

Leu Ile Gly Cys Arg Pro Pro Ile Gly Glu His Trp Gly Lys Gly Thr
                165                 170                 175

Pro Cys Asn Ala Asn Gln Val Lys Ala Gly Glu Cys Pro Pro Leu Glu
            180                 185                 190

Leu Leu Asn Thr Val Leu Gln Asp Gly Asp Met Val Asp Thr Gly Phe
        195                 200                 205

Gly Ala Met Asp Phe Thr Thr Leu Gln Ala Asn Lys Ser Asp Val Pro
    210                 215                 220

Leu Asp Ile Cys Ser Ser Ile Cys Lys Tyr Pro Asp Tyr Leu Lys Met
225                 230                 235                 240

Val Ser Glu Pro Tyr Gly Asp Met Leu Phe Phe Tyr Leu Arg Arg Glu
                245                 250                 255

Gln Met Phe Val Arg His Leu Phe Asn Arg Ala Gly Thr Val Gly Glu
            260                 265                 270

Thr Val Pro Ala Asp Leu Tyr Ile Lys Gly Thr Thr Gly Thr Leu Pro
        275                 280                 285

Ser Thr Ser Tyr Phe Pro Thr Pro Ser Gly Ser Met Val Thr Ser Asp
    290                 295                 300

Ala Gln Ile Phe Asn Lys Pro Tyr Trp Leu Gln Arg Ala Gln Gly His
305                 310                 315                 320

Asn Asn Gly Ile Cys Trp Ser Asn Gln Leu Phe Val Thr Val Val Asp
                325                 330                 335

Thr Thr Arg Ser Thr Asn Met Ser Val Cys Ser Ala Val Ser Ser Ser

            340                 345                 350

Asp Ser Thr Tyr Lys Asn Asp Asn Phe Lys Glu Tyr Leu Arg His Gly
        355                 360                 365

Glu Glu Tyr Asp Leu Gln Phe Ile Phe Gln Leu Cys Lys Ile Thr Leu
    370                 375                 380

Thr Ala Asp Val Met Thr Tyr Ile His Ser Met Asn Pro Ser Ile Leu
385                 390                 395                 400

Glu Asp Trp Asn Phe Gly Leu Thr Pro Pro Pro Ser Gly Thr Leu Glu
                405                 410                 415

Asp Thr Tyr Arg Tyr Val Thr Ser Gln Ala Val Thr Cys Gln Lys Pro
            420                 425                 430

Ser Ala Pro Lys Pro Lys Asp Asp Pro Leu Lys Asn Tyr Thr Phe Trp
        435                 440                 445

Glu Val Asp Leu Lys Glu Lys Phe Ser Ala Asp Leu Asp Gln Phe Pro
    450                 455                 460

Leu Gly Arg Lys Phe Leu Leu Gln Ala Gly Leu Lys Ala Arg Pro Asn
465                 470                 475                 480

Phe Arg Leu Gly Arg Arg Ala Ala Pro Ala Ser Thr Ser Lys Lys Ser
                485                 490                 495

Ser Thr Lys Arg Arg Lys Val Lys Ser
            500                 505